# EUROPEAN PATENT APPLICATION

(11) **EP 3 916 042 A1**
(43) Date of publication of application: **01.12.2021**
(21) Application number: 21175371.0
(22) Date of filing: 21.05.2021
(51) Int. Cl.: C08K 3/015, A01N 59/16, B60N 3/00, B60R 99/00, C08K 5/00, C09D 5/00, C09D 5/14, C09D 7/61, C09D 7/63, C09D 5/18, C09D 7/48, C08K 3/014, C08J 7/04

(54) **DISINFECTING TOUCH SURFACES WITHIN AIRCRAFT**

(30) Priority: 21.05.2020 US 202063028195 P; 21.05.2020 US 202063028199 P; 21.05.2020 US 202063028211 P; 21.05.2020 US 202063028206 P
(71) Applicant: B/E Aerospace, Inc., Winston-Salem, NC 27105 (US); Goodrich Corporation, Charlotte, NC 28217-4578 (US)
(72) Inventor: POTEET, Steven, Ashland, MA 01721 (US); McCONNELL, David Charles, Winston-Salem, NC 27006 (US); MARTZ, Thomas, Winston-Salem, NC 27106 (US); ST. ROCK, Brian, Andover, CT 06232 (US); PUJAR, Vijay V., Rancho Santa Fe, CA 92067-5010 (US); SMITH, Blair, South Windsor, CT 06074 (US); MOREAU, Claude J., Vernon, CT 06066 (US)
(74) Representative: Dehns

(57) **Abstract**

A component is provided having a component body defining at least one touch point surface. The component body includes an additive material that prevents degradation of a material of the component body in response to a cleaning agent.

## Description

### BACKGROUND

Embodiments of the disclosure relate to the sterilization of an aircraft, and more specifically, to one or more components within the aircraft that can withstand repeated sterilization system.

Processes for cleaning and decontaminating aircraft after each flight are typically determined by an airline. Such processes typically include picking up garbage, and wiping down surfaces such as tray tables, arm rests, and seat belt buckles. However, existing processes may not be sufficient to kill bacteria or viruses present within all of the passenger occupied areas of an aircraft. Accordingly, the use of other sterilization processes, such as electrostatic or chemical cleaning and the use of ultraviolet light are currently being explored.

### BRIEF SUMMARY

According to an embodiment, a component is provided having a component body defining at least one touch point surface. The component body includes an additive material that prevents degradation of a material of the component body in response to a cleaning agent.

In addition to one or more of the features described above, or as an alternative, in further embodiments the cleaning agent is one of ultraviolet light and radiation.

In addition to one or more of the features described above, or as an alternative, in further embodiments the additive material is one or more of ultraviolet stabilizers, ultraviolet absorbers, hindered amine light stabilizers, titanium dioxide, or zinc dioxide.

In addition to one or more of the features described above, or as an alternative, in further embodiments the additive material is embedded within the component body.

In addition to one or more of the features described above, or as an alternative, in further embodiments the additive material is coating in overlapping arrangement with one or more surfaces of the component body.

In addition to one or more of the features described above, or as an alternative, in further embodiments the additive material is applied via one of atomic layer deposition, chemical vapor deposition, physical vapor deposition, dip coating, and spraying.

In addition to one or more of the features described above, or as an alternative, in further embodiments the component is mounted within a cabin of an aircraft.

In addition to one or more of the features described above, or as an alternative, in further embodiments the component body further comprises an antimicrobial material operable to deactivate a microbe arranged in contact with a surface of the touch point.

In addition to one or more of the features described above, or as an alternative, in further embodiments the antimicrobial material is embedded within the component body.

In addition to one or more of the features described above, or as an alternative, in further embodiments the antimicrobial material is a coating in overlapping arrangement with one or more surfaces of the component body.

In addition to one or more of the features described above, or as an alternative, in further embodiments the antimicrobial material is one of copper and silver.

In addition to one or more of the features described above, or as an alternative, in further embodiments the antimicrobial material is a photocatalytic material.

In addition to one or more of the features described above, or as an alternative, in further embodiments the photocatalytic material is doped.

In addition to one or more of the features described above, or as an alternative, in further embodiments photocatalytic activity of the photocatalytic material is enhanced by the presence of at least one of silver, zeolites or montmorillonite.

In addition to one or more of the features described above, or as an alternative, in further embodiments the component body further comprises a fire resistant material.

In addition to one or more of the features described above, or as an alternative, in further embodiments at least one of the antimicrobial material and the fire resistant material includes copper, silver, quaternary ammonium compounds, zinc pentathione, organophosphorous compounds, or nano-platelets such as clays, h-boron nitride, or graphene.

In addition to one or more of the features described above, or as an alternative, in further embodiments the fire resistant material is different than the antimicrobial material.

In addition to one or more of the features described above, or as an alternative, in further embodiments the fire resistant material and the antimicrobial material are the same.

### BRIEF DESCRIPTION OF THE DRAWINGS

The following descriptions should not be considered limiting in any way. With reference to the accompanying drawings, like elements are numbered alike:
FIG. 1 is a perspective view of a portion of an interior of a passenger vehicle;
FIG. 2 is a schematic view of a touch point having an additive material applied as a coating according to an embodiment;
FIG. 3 is a schematic view of a component body defining a touch point having another coating according to an embodiment; and
FIG. 4 is a schematic view of a component body defining a touch point having a coating according to another embodiment.

### DETAILED DESCRIPTION

A detailed description of one or more embodiments of the disclosed apparatus and method are presented herein by way of exemplification and not limitation with reference to the Figures.

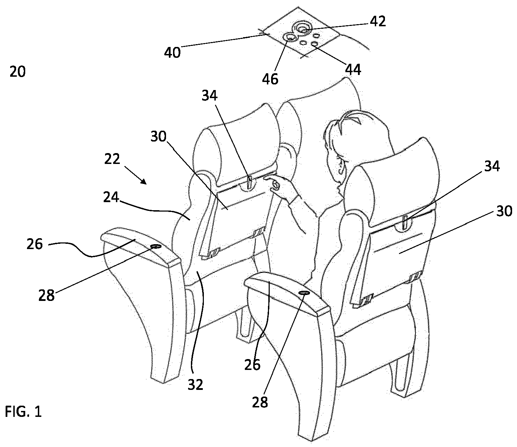

With reference now to FIG. 1, an example of a portion of an interior 20 of a passenger vehicle, such as a cabin of an aircraft for example, is illustrated in more detail. Within the cabin 20, there are several components or areas that a passenger is likely to touch or come into contact with. Surfaces of these components or areas, referred to herein as "touch points" may be a primary focus during cleaning or sterilization operations to prevent the transmission of bacteria, viruses, or other microbes.

As shown, the cabin 20 includes a plurality of seats 22 which are typically occupied by passengers during flight. In the illustrated, non-limiting embodiment, the passenger seats 22 have a seat back 24 and arm rests 26 disposed on opposing sides of the seat back 24. The seat 22 may include a reclining button or lever 28 operable to allow a user to transform the seat back 24 between an upright position and a reclined position. Although not shown, two portions of a seat belt may be affixed about the passenger via a buckle to restrict movement of the passenger relative to the seat 22. Further, a tray table 30 movable between a retracted and an extended position is mounted at the back 32 of each passenger seat 22 for use by the passenger seated in the next row. Each of the tray table 30, the latch 34 used to retain the tray table 30 in the retracted position, the arm rests 26, the seat belt, and the reclining button 28 may be considered a touch point associated with a seat 22. Although not shown, a magazine pouch, such as typically located beneath the tray table 30, may be considered another touch point associated with the seat 22.

Additionally, one or more passenger service units 40 are mounted within the cabin 20, such as above the passenger seats. In an embodiment, the cabin includes a plurality of passenger service units 40 and each passenger service unit 40 is associated with at least one of the plurality of passenger seats 22, such as a single row of passenger seats for example. The passenger service units 40 may include at least one light source or reading light 42, an attendant call button 44, and a ventilation supply nozzle 46. However, a passenger service unit 40 having any configuration of components or inputs thereon are within the scope of the disclosure. The passenger service unit 40 and/or the components mounted thereto may also be considered touch points within the cabin 20.

Other touch points within the cabin 20 that are not shown in the FIGS. include, but are not limited to, the overhead luggage bins, the latches for accessing these bins, window shades, volume control buttons on the arm rests, ear plug or headphone jack, the display screen mounted at the seat back including the associated channel and volume controls, and the folding seats for flight attendants. Other examples include hand rails used to board the aircraft or during flight, elevator buttons, emergency door handles, and window escape handles. Further, within the one or more galleys of the cabin 20, the food carts, oven, microwave, and refrigerator may be considered touch points, and similarly, within the lavatories, any of the toilet components, the sink and its respective control component (faucets including handles and buttons that control the flow of hot and cold liquid), soap dispenser, towel dispenser, or door latch, may be considered touch points.

The touch points illustrated and described herein are intended as examples only, and it should be understood that any surface, area, or component that is contacted by one or more persons during transport or operation of the aircraft may be considered a touch point. Further, it should be understood that although the touch points are illustrated and described herein with respect to an aircraft, any vehicle, building, or area having one or more surfaces that experience significant human traffic and contact are within the scope of the disclosure. Examples of other vehicles or areas include, but are not limited to a ground transportation vehicle, such as a train, subway, monorail, trolley, and automobile, as well as an elevator, people mover, cruise ship, and amusement park ride.

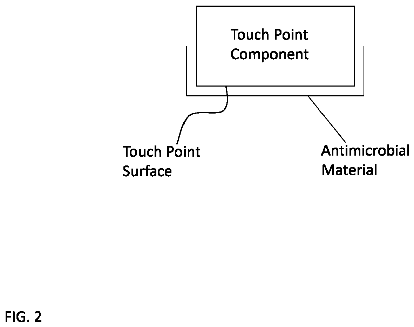

Some or all of the touch points of an aircraft are typically disinfected during regular cleanings, such as between scheduled flights of the aircraft for example. However, there is a need to sterilize any microbes, bacteria, or viruses that are present on touch points between cleanings to minimize the potential for transmission of microbes. In an embodiment, the materials commonly used to form these touch points, such as polymer for example, may be modified to include antimicrobial properties capable of deactivating or neutralizing any microbes, including bacteria and viruses, that contact the material (see FIG. 2). Examples of suitable materials that could be incorporated into the touch points to provide antimicrobial properties include, but are not limited to, copper and silver for example. As a result, when a microbe, such as a virus or bacteria is transmitted to a surface of the contact point, the antimicrobial material will neutralize of kill the microbe, thereby preventing transfer of the microbe.

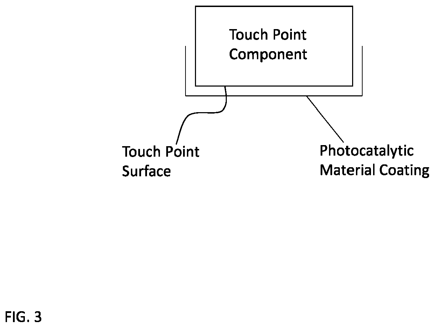

Alternatively, or in addition, with reference to FIG. 3, a photocatalytic material may be incorporated into or applied to the component body as an antimicrobial material. As described herein, a photocatalytic material is configured to deactivate or neutralize a microbe arranged in contact with the photocatalytic material when the photocatalytic material is activated by a light having a specific wavelength. Suitable wavelengths include ultraviolet light, visible light, or any other desired wavelength. Any photocatalytic material exhibiting a band gap which can be excited by incident light to create positive holes and electrons of sufficient energy to create oxidizing and reducing radicals may be suitable. Examples of photocatalytic materials include, but are not limited to, titanium dioxide (TiO2) and doped titanium dioxide, and zinc oxide (ZnO), for example. The titanium dioxide may be doped, for example, with sulfur or nitrogen, to alter the band gap energy so that the surface of the photocatalytic material may be activated by the incidence of visible light

Further, in an embodiment, the photocatalytic activity of the photocatalytic material may be enhanced by combining the photocatalytic material with other materials. Examples of such other materials include but are not limited to, silver, zeolites and layered silicate materials such as montmorillonite. For example, montmorillonite may help to absorb light and enhance the effectiveness of titanium dioxide in creating oxidizing and reducing radicals that help to deactivate microbes.

These antimicrobial materials may be embedded into the material used to form a component that defines a touch points, such as during an extrusion or injection molding process for example. For example, the antimicrobial material can be embedded into a filament material prior to extrusion. Similarly, in embodiments where the component body is formed via an additive manufacturing process, nanoparticles of the antimicrobial material can be mixed in with the base resin material or other materials used to form the individual layers of the component body. Examples of suitable additive manufacturing processes include but are not limited to stereolithography, selective laser sintering, fused deposition modeling, fused filament fabrication. Formation via such additive manufacturing processes should not alter the fire, smoke, and toxicity properties of the component body.

Alternatively, or in addition, these additives antimicrobial materials may be applied as a coating, such as a clear top coat for example, overlapping one or more surfaces of the component that defines the touch points after manufacture of the component. In embodiments where the photocatalytic material is applied as a coating extending over one or more surfaces that define the touch point of the component body, the coating may be a thin layer of material, for example having a thickness less than or equal to 100 nm. In other embodiments, the coating may be thicker, having a thickness of up to or even greater than 100 micrometers. Further, any suitable method for applying the coating material to the one or more surface of the component is contemplated herein. Examples of such methods include, but are not limited to atomic layer deposition, chemical vapor deposition, and physical vapor deposition, dip coating, spraying or painting.

In an embodiment, the light source used as the catalyst to activate the photocatalytic material to "clean" a touch point is provided by a passenger. For example, a passenger may shine the light emitted by a cell phone when in "flashlight" mode over one or more exposed surfaces of the touch point. This allows a user to sterilize the surfaces of the touch point before contacting the touch point. Alternatively, or in addition, one or more light sources mounted within the cabin 20 may be energized to initiate a cleaning operation. The use or one or more light sources mounted within the cabin may occur in cycles scheduled at a predetermined time interval. This use of one or more light sources onboard the aircraft may occur when the aircraft is in any flight condition, and may even occur regardless of whether passengers are on board depending on the wavelength of the light emitted by the light source being used.

The material with antimicrobial properties as described above requires activation, such as by the application of energy or a light for example, to neutralize any microbes on the surface of the material. In another embodiment, the material having antimicrobial properties is capable of automatically deactivating or neutralizing microbes arranged in contact therewith, without any external stimulus. In such applications, any microbes transmitted to the touch point will be neutralized upon contact with the antimicrobial material, thereby preventing transfer of the microbes to another surface or to a passenger.

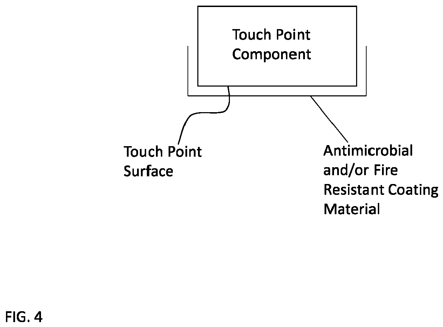

With reference now to FIG. 4, a material used to form the component that defines a touch point may be modified to include not only antimicrobial properties, but also flame resistant or flame retardant properties. Examples of suitable materials that could be used to provide both antimicrobial and fire resistant properties include, but are not limited to, copper, silver, quaternary ammonium compounds, zinc pentathione, organophosphorous compounds, or nano-platelets such as clays, h-boron nitride, or graphene. As a result, when a microbe, such as a virus or bacteria is transmitted to a surface of the touch point, the antimicrobial material will neutralize of kill the microbe, thereby preventing transfer of the microbe.

The additives or materials configured to provide antimicrobial and fire resistant properties may be embedded into the material used to form the body or structure of the components that define the touch points, such as during an extrusion or injection molding process for example. In such embodiments, the antimicrobial and fire resistant materials may be embedded in the entire component body, or only a portion thereof. For example, the antimicrobial and fire resistant material may be located only adjacent to the exposed surfaces that define the touch point for example. Alternatively, or in addition, these additives may be applied as a coating, such as a clear top coat for example, overlapping one or more surfaces of the components that define the touch points after the components have been manufactured.

As previously described, the interior of the aircraft including one or more of the touch points may be disinfected via regular cleanings. Such cleaning operations may use ultraviolet light or another form of radiation to disinfect surfaces. In an embodiment, the materials used to form the touch points are modified to prevent degradation thereof in response to continued or regular exposure to a cleaning agent like ultraviolet light or radiation, or alternatively, a chemical solution. This modification to prevent degradation may be alternative to or in addition to the modification to the materials to include antimicrobial and/or fire resistant properties as previously described herein. Examples of materials that may be added to the material of a touch point to prevent degradation include, but are not limited to, one or more of ultraviolet stabilizers, ultraviolet absorbers, hindered amine light stabilizers, titanium dioxide, or zinc dioxide. These additives may be embedded into the material used to form the components that define the touch points, such as during an extrusion or injection molding process for example. Alternatively, these additives may be applied as a coating, such as a clear top coat for example, overlapping one or more surfaces of the components that define the touch points after the components have been manufactured.

Modifying the material composition of the component bodies that define the touch points helps maintain the surfaces of the touch points free from microbes. As a result, the potential for transmission of microbes and spores between passengers is reduced, while meeting the strict fire, smoke, and toxicity requirements associated with certain applications, such as aircraft. The antimicrobial, photocatalytic material, and/or fire resistant materials may be compatible with other disinfection solutions, thereby providing an additional layer of defense against contamination of the touch points while also preventing degradation of the structure that defines the touch point.

The term "about" is intended to include the degree of error associated with measurement of the particular quantity based upon the equipment available at the time of filing the application.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

While the present disclosure has been described with reference to an exemplary embodiment or embodiments, it will be understood by those skilled in the art that various changes may be made and equivalents may be substituted for elements thereof without departing from the scope of the present disclosure. In addition, many modifications may be made to adapt a particular situation or material to the teachings of the present disclosure without departing from the essential scope thereof. Therefore, it is intended that the present disclosure not be limited to the particular embodiment disclosed as the best mode contemplated for carrying out this present disclosure, but that the present disclosure will include all embodiments falling within the scope of the claims.

## Claims

1. A component comprising:
a component body defining at least one touch point surface, the component body including an additive material that prevents degradation of a material of the component body in response to a cleaning agent.

2. The component of claim 1, wherein the cleaning agent is one of ultraviolet light and radiation.

3. The component of claim 1 or 2, wherein the additive material is one or more of ultraviolet stabilizers, ultraviolet absorbers, hindered amine light stabilizers, titanium dioxide, or zinc dioxide.

4. The component of any preceding claim, wherein the additive material is embedded within the component body.

5. The component of any preceding claim, wherein the additive material is coating in overlapping arrangement with one or more surfaces of the component body.

6. The component of any preceding claim, wherein the additive material is applied via one of atomic layer deposition, chemical vapor deposition, physical vapor deposition, dip coating, and spraying.

7. The component of any preceding claim, wherein the component is mounted within a cabin (20) of an aircraft.

8. The component of any preceding claim, wherein the component body further comprises an antimicrobial material operable to deactivate a microbe arranged in contact with a surface of the touch point.

9. The component of claim 8, wherein the antimicrobial material is embedded within the component body.

10. The component of claim 8 or 9, wherein the antimicrobial material is a coating in overlapping arrangement with one or more surfaces of the component body.

11. The component of claim 8, 9, or 10, wherein the antimicrobial material is one of copper and silver.

12. The component of any of claims 8 to 11, wherein the antimicrobial material is a photocatalytic material; optionally wherein the photocatalytic material is doped.

13. The component of claim 12 or 13, wherein photocatalytic activity of the photocatalytic material is enhanced by the presence of at least one of silver, zeolites or montmorillonite.

14. The component of any of claims 8 to 13, wherein the component body further comprises a fire resistant material; optionally wherein the fire resistant material is different than the antimicrobial material..

15. The component of claim 14, wherein at least one of the antimicrobial material and the fire resistant material includes copper, silver, quaternary ammonium compounds, zinc pentathione, organophosphorous compounds, or nano-platelets such as clays, h-boron nitride, or graphene.
